(19) **Europäisches Patentamt / European Patent Office / Office européen des brevets**

(11) **EP 2 097 359 B1**

(12) **EUROPEAN PATENT SPECIFICATION**

(45) Date of publication and mention
of the grant of the patent:
**31.08.2011  Bulletin 2011/35**

(51) Int Cl.:
*C07C 5/22* (2006.01)        *C10G 49/00* (2006.01)
*C10G 45/58* (2006.01)       *C10G 35/04* (2006.01)

(21) Application number: **07867506.3**

(22) Date of filing: **19.11.2007**

(86) International application number:
**PCT/US2007/024120**

(87) International publication number:
**WO 2008/063584 (29.05.2008 Gazette 2008/22)**

(54) **NON-REFLUXING REACTOR STRIPPER**

REAKTORSTRIPPER OHNE RÜCKFLUSS

COLONNE DE STRIPPING SANS REFLUX DE RÉACTEUR

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority:  **20.11.2006  US 602020**

(43) Date of publication of application:
**09.09.2009  Bulletin 2009/37**

(73) Proprietor: **Lummus Technology Inc.
Bloomfield, NJ 07003 (US)**

(72) Inventor: **ARNOLD, Stephen Craig
Mountain Lakes, NJ 07046 (US)**

(74) Representative: **Thoma, Michael et al
Lorenz - Seidler - Gossel
Widenmayerstrasse 23
80538 München (DE)**

(56) References cited:
**WO-A-00/15319        DE-A1- 10 243 444
US-A- 5 830 345        US-A- 5 948 948**

## Description

### 1. Field of the Invention

The present invention relates to an apparatus for the catalytic distillation of organic compounds, more particularly to a process for catalytically treating a feed stream containing at least one organic compound.

### 2. Background of the Art

The use of a catalyst in a distillation column to concurrently carry out chemical reactions and separate the reaction products has been practiced for some time. This use of a catalytic distillation column reactor lends itself particularly well for reversible reactions in the liquid phase. The combination is useful due to quick separation of the reaction products from the reactants in the liquid phase by fractional distillation resulting from boiling point differences. Thus, the reverse reaction is suppressed. It is also useful for liquid phase reactions where one or more compounds produced causes an adverse impact if it remains in the reaction mixture. Therefore, it is beneficial to remove such components quickly from the reaction mixture.

The removal of selected components of the reaction mixture from the reaction zone in catalytic distillation is rapid when the relative volatilities of the components are significantly different. However, this advantage can be lessened to a significant extent by the return of the selected components into the reaction zone by reflux of the fractionation returning higher volatility components to the reaction zone, or by the reboiler returning lower volatility components to the reaction zone. This is especially the case when the relative volatilities of the components are close to 1.0.

In an exemplary application, increasingly stringent regulations will put pressure on refiners to remove aromatics from motor fuels. Skeletal isomerization of straight chain hydrocarbons into high-octane, branched paraffins is an effective route to compensate for the octane loss associated with aromatics removal. It is desirable to convert n-heptanes or mono-branched $C_7$ paraffins into di- or tri-branched $C_7$ paraffins, which can be used to provide good contribution to the octane of motor fuels. For example, Table 1 below illustrates the research octane number (RON) for the $C_7$ paraffins.

Table 1

| Heptane Isomer | RON | B.P., °F |
|---|---|---|
| 2,2,3-Trimethylbutane | 112.1 | 177.59 |
| 2,2-Dimethylpentane | 92.8 | 174.55 |
| 2,4-Dimethylpentane | 83.1 | 176.90 |
| 3,3-Dimethylpentane | 80.8 | 186.92 |
| 2,3-Dimethylpentane | 91.1 | 193.61 |

(continued)

| Heptane Isomer | RON | B.P., °F |
|---|---|---|
| 2-Methylhexane | 42.4 | 194.09 |
| 3-Methylhexane | 52.0 | 197.33 |
| 3-Ethylpentane | 65.0 | 200.26 |
| n-Heptane | 0 | 209.17 |

However, isomerization of n-heptane has proven to be difficult for a number of reasons. First, the volatility of the $C_7$ isomers is close to that of n-heptane (see Table 1), making separation more difficult. Second, conventional isomerization catalysts also tend to result in undesirable cracking of the $C_7$ compounds. What is needed is a distillative reaction system which provides faster separation and removal of the desired isomers from the distillative reaction system vessel.

A process for catalytically treating a feed stream containing an organic compound in a distillative reaction system is known, for example, from DE-A-10243444, wherein the oligomerization of $C_4$ hydrocarbon mixtures is effected in two reactive distillation columns in series. More particularly, the product stream is sent into a second distillation column having another catalyst bed and undergoes a second cracking step, wherein the second cracking product stream is then returned to the first distillation column.

Furthermore, document WO-A-0015319 discloses a process for the hydrodesulfurization of naphtha in a distillation column reactor having a stripper section, wherein a product flowline is sent through a condenser, then through a separator, then through another condenser and the light product line is then returned to the uppermost portion of the distillation column.

It is an objective of the present invention to provide an improved process for catalytically treating a feed stream containing at least one organic compound, wherein undesirable cracking of the products is minimized.

According to the present invention, this objective is achieved by a process as defined in claim 1. Preferred embodiments of the invention are laid down in the dependent claims.

### SUMMARY OF THE INVENTION

A process for catalytically treating a feed stream containing at least one organic compound is provided herein. The process comprises (a) providing a distillative reaction system having at least an upper reaction section positioned at a top portion of the distillative reaction system and a reboiler and/or gas stripping section for vaporizing at least a portion of a bottom stream and returning the vaporized portion of the bottom stream to a bottom portion of the distillative reaction system; (b) introducing an organic feed stream into the distillative reaction system below the uppermost reaction section; and, (c) removing an overhead product stream from a portion of the distillative reaction system above the uppermost reaction section without substantial reflux or recycling of any product-containing stream or feeding any other compounds that are undesirable to be refluxed, into the uppermost reaction section. Optionally, a gaseous reactant feed stream can be introduced into the distillative reaction system or below the uppermost reaction section

The process advantageously prevents the return of high volatility components to the uppermost portion of the reaction section and the resulting undesirable cracking of the desired product.

Various other features, aspects, and advantages of the invention will become more apparent with reference to the following detailed description of exemplary embodiments and appended claims.

## BRIEF DESCRIPTION OF THE DRAWINGS

Fig. 1 is a schematic process flow diagram of an exemplary distillative reaction system which is no embodiment of the invention.

Fig. 1A is a schematic process flow diagram of another exemplary distillative reaction system which is no embodiment of the invention.

Fig. 1B is a schematic process flow diagram of another exemplary distillative reaction system which is no embodiment of the invention.

Fig. 2 is a schematic process flow diagram of an exemplary embodiment of the invention.

Fig. 3 is a schematic process flow diagram of another exemplary embodiment of the invention.

Fig. 3A is a schematic process flow diagram of another exemplary embodiment of the invention.

Fig. 4 is a schematic process flow diagram of yet still another exemplary distillative reaction system which is no embodiment of the invention.

Fig. 4A is a schematic process flow diagram of another exemplary embodiment of the invention.

Fig. 4B is a schematic process flow diagram of another exemplary distillative reaction system which is no embodiment of the invention.

Fig. 5 is a schematic process flow diagram of yet another exemplary embodiment of the invention.

Fig. 6 is a schematic process flow diagram of still another exemplary embodiment of the invention.

Fig. 6A is a schematic process flow diagram of yet still another exemplary embodiment of the invention.

Fig. 7 is a schematic process flow diagram of yet another exemplary embodiment of the invention.

## DETAILED DESCRIPTION OF EXEMPLARY EMBODIMENTS

In the description below, similar numerals indicate similar features of the invention.

In accordance with an exemplary embodiment of the invention, a process for catalytically treating a feed stream containing at least one organic compound is provided which comprises providing a distillative reaction system having a reaction section positioned at a top portion of the distillative reaction system and a reboiler and/or gas stripping section for vaporizing at least a portion of a bottom stream and returning the vaporized portion of the bottom stream to a bottom portion of the distillative reaction system, introducing an organic feed stream into the distillative reaction system below the uppermost reaction section, optionally introducing a gaseous reactant feed stream into the distillative reaction system below the uppermost portion of the reaction section, and removing an overhead product stream from a portion of the distillative reaction system above the reaction section without refluxing any substantial portion of the overhead product stream or any other liquid stream that might recycle the desired products or feeding any other compounds that are undesired to be refluxed into the uppermost portion of the reaction section.

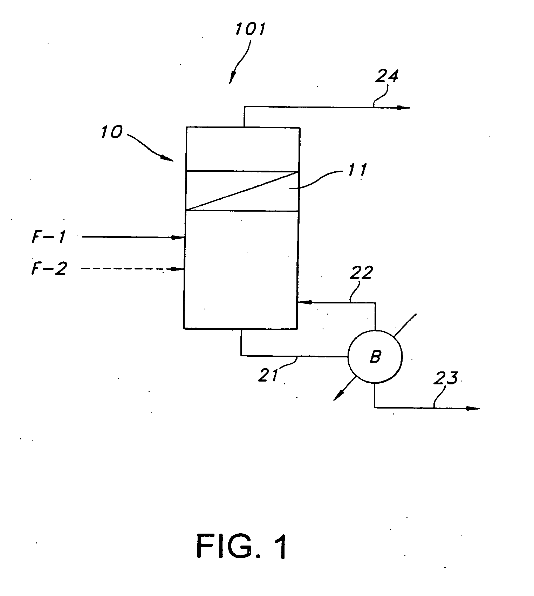

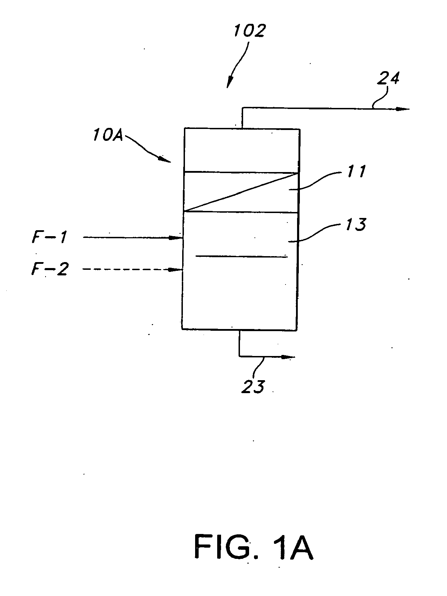

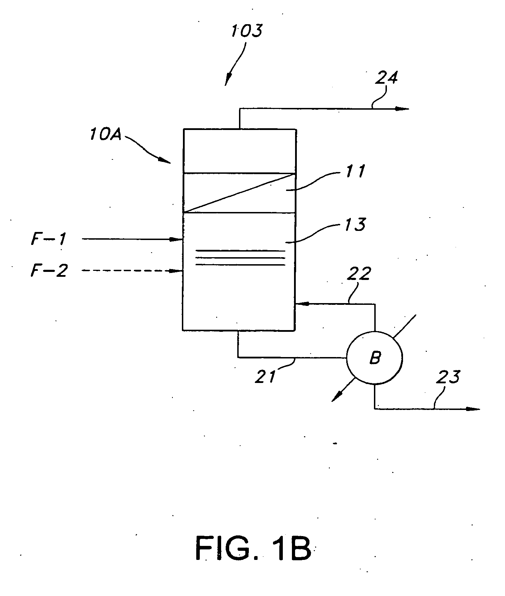

Referring now to the process flow diagram of FIG. 1, an exemplary distillative reaction system which is no embodiment of the invention includes system 101 comprising a distillative reaction system 10 for the isomerization of at least one paraffin. Distillative reaction system 10 includes a reaction section 11 without any substantial reflux above the reaction section 11. The reaction section 11 includes at least one catalyst bed.

Suitable catalysts for this exemplary application can be any conventional catalysts known in the art which are appropriate for the desired reaction. For example, a useful catalyst for isomerization of a paraffin corresponds to the formula

$$R_1/R_4/R_2\text{-}R_3$$

wherein $R_1$ is a metal, metal alloy, or bimetallic system; $R_2$ is any metal dopant; $R_3$ is a metallic oxide or mixture of any metallic oxide; $R_4$ is $WO_x$, $MoO_x$, $SO_4^{-2}$ or $PO_4^{-3}$ and x is 2 or 3, or any fractional number therebetween. A catalyst of this type is disclosed and described in U.S. Patent No. 6,767,859, the entire contents of which are incorporated herein by reference.

The catalyst may be in any suitable form such as powder, pellets, rings, extrudates, spheres, and the like. The catalyst bed in the reaction section 11 is a fixed bed of catalyst in distillative "packaging".

A hydrocarbon feed including at least one paraffin capable of isomerization is introduced into the distillative reaction system 10 via feed stream F-1 and/or F-2. The hydrocarbon feed including at least one paraffin can be introduced into the distillative reaction system 10 as a liquid and/or vapor. The paraffin may be selected from the group consisting of n-heptane, 2-methyl hexane, 3-methyl hexane, 3-ethyl pentane, or any of the Cs, $C_6$, $C_7$, $C_8$, and/or $C_9$, $C_{10}$, $C_{11}$ normal or mono-substituted paraffins in this example of isomerization to obtain octane contribution for motor fuels.

In the isomerization example, a gaseous reactant feed $H_2$ (which may also include other constituents), which also serves as a stripping gas, is introduced into the distillative reaction system 10 via feed stream F-2 and/or F-1.

A bottom stream 21 is drawn off a lower portion of the distillative reaction system 10 and sent to a reboiler B wherein at least a portion of the bottom stream is vaporized and returned to the lower portion of the distillative reaction system 10 via line 22. An unvaporized portion of the bottom stream 21 may be drawn off as liquid via line 23.

An overhead stream 24 is drawn off the top portion of the distillative reaction system 101. However, there is no reflux of any substantial portion of overhead stream 24. That is, in order to avoid hindering the exit of the higher volatility desired products, and to minimize any undesirable reactions, e.g., cracking, of the desired product, substantially none of the overhead stream 24 is returned to the top of the distillative reaction system nor is there any substantial cooling to cause condensation and internal reflux nor any substantial other liquid stream fed above reaction section 11 in a way that would cause reflux down into reaction section 11.

The isomerization process typically converts n-paraffin, such as n-heptane, to mono-branched isoheptanes. The mono-branched isoheptanes are further isomerized to di- and tri-branched $C_7$ paraffins.

By way of example, the feed stream at F-1 and/or F-2 is n-heptane (plus accompanying compounds). The bottom stream 21 contains unreacted n-heptane plus a very small amount of heavy reaction by-products plus any feed compounds that were as heavy as n-heptane or heavier (e.g., dimethyl cyclopentanes, methyl cyclohexane, toluene, C8+ hydrocarbons), plus an amount of hydrocarbons a little lighter than n-heptane (e.g., methyl hexanes). The reboiler returns stream 22 with the lighter compounds in stream 21 (i.e., the dimethyl cyclopentanes, methyl hexanes and lighter) including as much of the n-heptane as desired (a higher percentage being returned if the reboiling is increased), while sending away the heaviest compounds in stream 23. Stream 23 includes compounds heavier than n-heptane (heavy reaction by-products, C8+hydrocarbons, toluene, methyl cyclohexane), some amount of n-heptane and even some amount of methyl hexanes (and ethyl pentane and dimethyl cyclopentanes); in an extreme of "total reboil", stream 23 could have zero flow and stream 22 return all of stream 21 to the distillative reaction system 10. The lightest compounds travel up the distillative reaction system 10 and leave through stream 24; this includes the residual $H_2$, light cracked products (primarily propane and isobutane), C5-C7 isoparaffins (including the most desired multi-isoheptane products, which are sought to be removed quickly out the top of the distillative reaction system 10), any n-pentane, n-hexane, benzene and cyclohexane, plus some amount of methyl hexanes (and ethyl pentane and dimethyl cyclopentanes) and even some n-heptane. It can be seen from the boiling points listed in Table 1 for C7 paraffins that 2,3-dimethylpentane distills together with 2-methylhexane.

Carrying out the isomerization process in a distillative reaction system without substantial reflux of the overhead product or any other liquid stream that might recycle the desired products or any other compounds that are undesired to be refluxed, removes the products quickly, thereby preventing substantial undesirable cracking. While it may be desirable to return a portion of the products of the first reaction step (n-paraffin to mono-branched isoparaffins), this process provides better results for the second step (mono-branched to di- and tri-branched compounds), than if refluxing is performed.

Additionally, uncoupling the reaction section from an overhead reflux allows more flexibility to optimize the reaction conditions. For example, in a conventional process with both reflux and reboil, the percent of vaporization of the mixture in the heart of a fractionation column would be limited to the relatively narrow range that is inherent for the fractionation function - about 50%. Operation of the present invention, without substantial reflux, can be carried out at any vaporization level, e.g., 10% to 80% and, at the high vaporization level, e.g. 70% to 80%, can provide improved reaction results due to both (a) hydrodynamic benefits and (b) heightened transfer of the higher volatility components (e.g., the product isoheptane isomers) into the vapor phase and out of the reaction zone. The lower volatility components (e.g., the feed n-heptane) become more concentrated in the liquid phase, enhancing their reaction rather than that of the higher volatility components. A mixed phase system with high boiling is hydrodynamically advantageous (provides stronger reaction) relative to a quiescent mixed phase system due to enhanced mass transfer between the liquid and vapor phases and also within the liquid phase by the strong mixing caused by the turbulence. The reaction conditions such as pressure, temperature, ratio of different feeds, etc. can also be optimized without being constrained to match the distillation conditions.

Alternatively, as shown in Fig. 1A, system 102 includes a distillative reaction system 10A which possesses a gas stripping section 13 in lieu of reboiler B.

The stripping section 13 may include one or more trays such as bubble cap trays, sieve trays and the like. Suitable trays for distillation columns are well known in the art and can be employed in the invention. Alternatively, the stripping section 13 may be a bed packed with inert material such as ceramics or metal rings, saddles, pellets, structured packing, etc.

Stripping gas is introduced into the distillative reaction system 10A via feed stream F-2, with possible additional gas via feed stream F-1. A gaseous reactant feed may be introduced into the distillative reaction system 10A at one or more locations via feed stream F-2 and/or F-1. Preferably, substantially all of, or at least a portion of, the gaseous reactant feed is introduced into the distillative reaction system 10A at a bottom portion of, or below, the stripping section 13 to function as a stripping gas as well as an isomerization reactant.

One or more hydrocarbon feed stream, including at least one paraffin hydrocarbon, is introduced into the distillative reaction system 10A via feed stream F-1 and/or F-2. All or portions of the hydrocarbon feed stream(s) can be introduced into the distillative reaction system 10A at the top of , below, or in the middle of stripping section 13. The preferred position(s) of the hydrocarbon feed stream(s) depends on its (or their) composition. The feed stream(s) F-1 and F-2 can be introduced as liquid(s) and/or vapor(s).

Optionally, side stream(s), not shown, can be used to draw-off selected hydrocarbon component(s) from the distillative reaction system 10A at a position such as the top, middle and/or bottom of stripping zone 13.

Referring to Fig. 1B, system 103 includes distillative reaction system 10A with a stripping section 13 and also has a reboiler B.

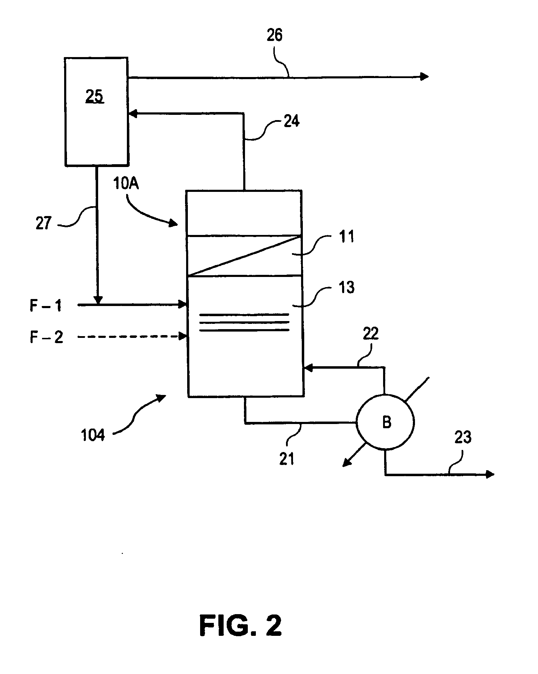

Referring to Fig. 2, in accordance with an exemplary embodiment of the invention system 104 includes distillative reaction system 10A with a stripping section 13 and/or a reboiler B (like any of systems 101 - 103), and further includes an overhead effluent separator 25 from which a first effluent 26 and a second effluent 27 are drawn off. The overhead stream 24 from the distillative reaction system 10A is sent to the overhead effluent separator 25 without any substantial reflux above the reaction section 11. The overhead effluent separator 25, as well as any of the effluent separators described below, can be a distillation column, molecular sieve, etc. The first effluent 26 contains the desired product. The second effluent 27 contains unconverted and/or partially converted paraffins. It is drawn off from the overhead effluent separator 25 and returned to the distillative reaction system 10A via feed stream F-1, below reaction section 11. Alternatively, the second effluent 27 is returned via feed stream F-2 or an F-3 (separate from other feeds).

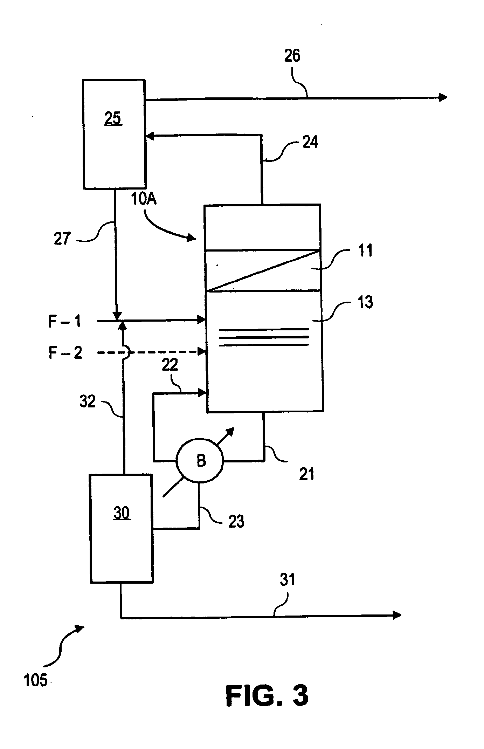

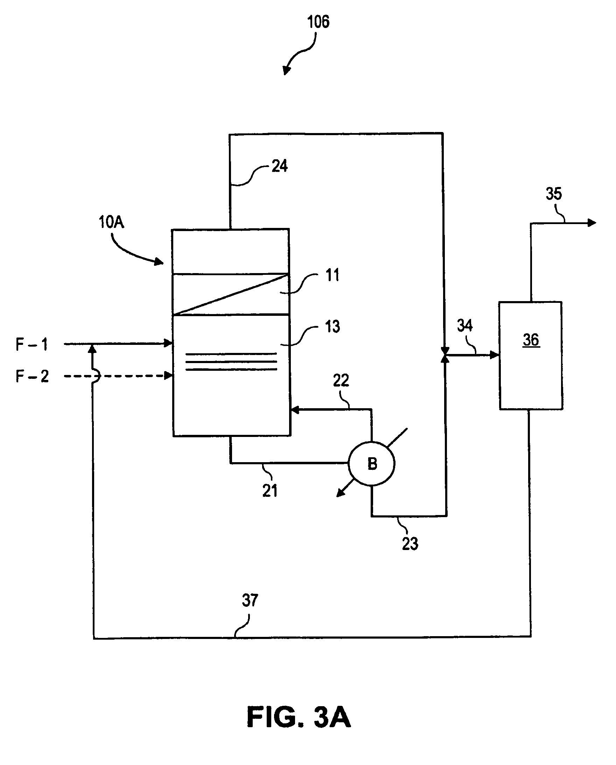

As shown in Fig. 3, in an alternative embodiment, system 105 includes distillative reaction system 10A, with a stripping section 13 and/or a reboiler B and with or without overhead effluent separator 25 (like any of systems 101 - 104), and further includes another (bottom) effluent separator 30 connected to the reboiler B via stream 23. The bottom effluent separator 30 includes a first effluent 31 and a second effluent 32. The first effluent 31 is low-volatility feed and/or byproduct compounds that need to be purged. The second effluent 32 contains low-volatility compound(s) that are sought to be recycled for further reaction and are returned to the distillative reaction system 10A via F-1 and/or F-2 (or an F-3).

Now referring to Fig. 3A, system 106 includes distillative reaction system 10A, reboiler B, and a single, combined effluent separator 36. Portions or all of overhead stream 24 from the distillative reaction system 10A and bottom stream 23 from the reboiler B are fed separately to effluent separator 36 and/or combined into stream 34 which is fed into effluent separator 36. A first effluent 35 from effluent separator 36 is sent to further processing. A second effluent 37 from effluent separator 36 is returned to the distillative reaction system 10A via F-1 and/or F-2 (or F-3).

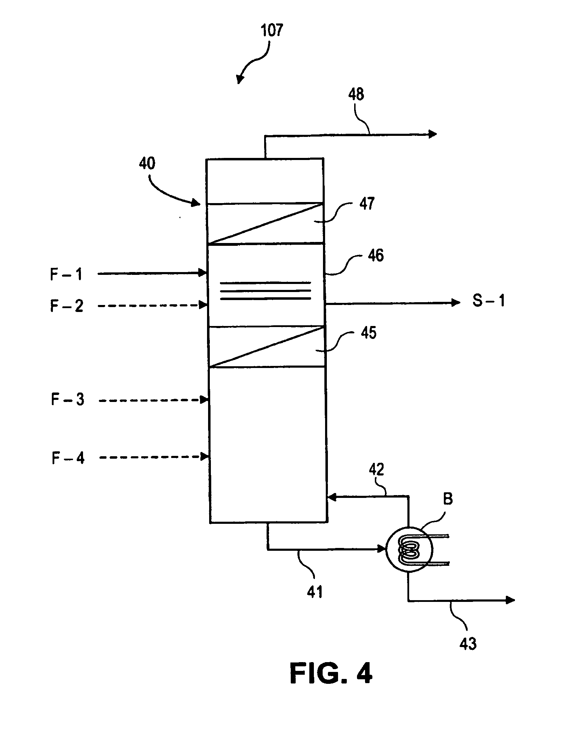

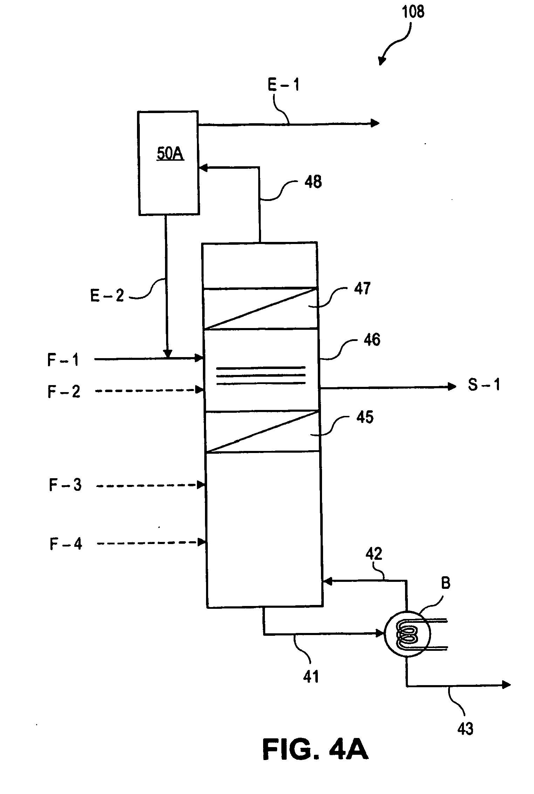

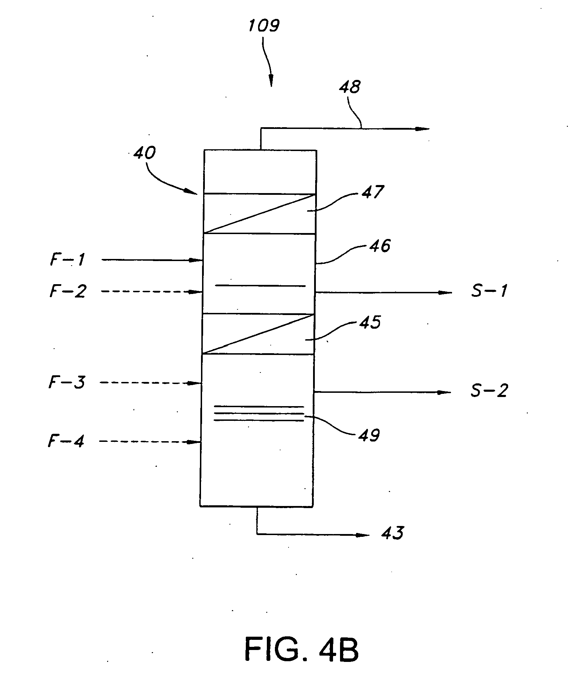

Referring now to Fig. 4, a process flow diagram for the isomerization of at least one paraffin depicts system 107 including a distillative reaction system 40 having an uppermost reaction section 47 and a lower reaction section 45. Each of the reaction sections 45 and 47 include at least one catalyst bed.

Suitable catalysts that can be employed in this exemplary distillative reaction system which is no embodiment have been described hereinabove. The catalyst bed in reaction section 45 and/or 47 is a fixed bed of catalyst in distillative "packaging". The catalysts for the two (or more) beds may be the same or may be different.

The distillative reaction system 40 may further include a distillation section 46 between reaction sections 45 and 47. The distillation section 46 may include one or more trays such as bubble cap trays, sieve trays and the like, or packing. Further, suitable trays or packing that may be employed herein are any trays or packing used in a catalytic distillation column that are well known in the art. Alternatively, there could be no distillation section 46 between the reaction sections 45 and 47.

A feed stream including at least one paraffin is introduced into the distillative reaction system 40 via feed streams F-1, F-2, F-3 and/or F-4. Feed F-1 might be introduced into distillative reaction system 40 above distillation section 46. Feed F-2 might be introduced into distillative reaction system 40 below, or at a midpoint of, distillation section 46 but above reaction section 45. Feeds F-3 and F-4 might be introduced into the distillative reaction system 40 below reaction section 45. The feed stream is introduced into the distillative reaction system 40 via F-1 and/or F-2, the lower reaction section 45 is a first-stage reactor, and the upper reaction section 47 is a second-stage reactor. The feed stream including the at least one paraffin is introduced into the distillative reaction system 40 as a liquid and/or vapor.

Optionally, one or more side stream S-1 can be used to draw off a desired product, or purge compound(s) that might otherwise accumulate in this or an adjacent zone, from the distillation section 46 of the distillative reaction system 40.

A gaseous reactant stream and/or stripping gas is introduced into the distillative reaction system 40 via feed stream F-1, F-2, F-3 and/or F-4. The gaseous reactant stream is introduced into the distillative reaction system 40 via F-3 and/or F-4 or alternatively via F-3 and/or F-4, with an additional amount via F-1 and/or F-2.

A bottom stream 41 is drawn off a lower portion of the distillative reaction system 40 and sent to a reboiler B wherein a portion of the bottom stream is vaporized and returned to the lower portion of the distillative reaction system 40 via line 42. An unvaporized portion of the bottom stream 41 is drawn off as a liquid via line 43.

An overhead stream 48 is drawn off a top portion of the distillative reaction system 40 with substantially no reflux. In other words, in order to minimize any undesirable reaction, e.g., cracking, substantially none of the overhead stream 48, or any other liquid stream that might recycle the desired products or any other compounds that are undesired to be refluxed, is returned to the top portion of the distillative reaction system 40 above upper reaction section 47, nor is there any substantial cooling to cause condensation and internal reflux nor any substantial other liquid stream fed above reaction section 47 in a way that would cause reflux down into reaction section 47.

An isomerization process utilizing system 107 typically converts a paraffin, such as n-heptane, to mono-branched isoheptanes (plus some additional isomers) in reaction section 45, and then further isomerizes the monobranched isoheptanes to di-and tri-branched $C_7$ paraffins in reaction section 47. Processing paraffin(s) in a distillative reaction system without substantial reflux of the overhead allows for quickly removing the product from the distillative reaction system, thereby minimizing any undesirable cracking of the products, especially the final, most isomerized and highest octane isoparaffins.

Now referring to Fig. 4A, system 108 includes distillative reaction system 40 with two reaction sections 45 and 47 and reboiler B (like system 107), and also has an overhead effluent separator 50A having a first effluent E1 and a second effluent E2. The overhead stream 48 from the distillative reaction system 40 is sent to the overhead effluent separator 50A. The first effluent E1 contains the desired product.

The second effluent E2 contains unconverted and/or partially converted paraffins. It is drawn off from the overhead effluent separator 50A and returned to the distillative reaction system 40 via F-1, F-2, F-3 and/or F-4.

Now referring to Fig. 4B, system 109 includes distillative reaction system 40A, with two reaction sections 45 and 47, and with gas stripping in lieu of the reboiler 41, illustrated as a bottom distillation section 49. Optionally, side stream S-1 and/or S-2 can be used to draw off from above or below the lower reaction section 45 component(s) that need purging as product(s) or in order to avoid building up.

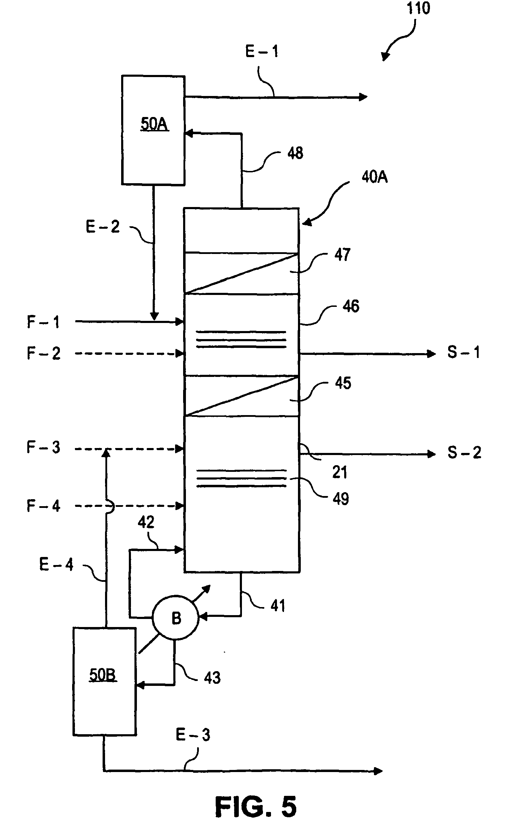

Fig. 5 is a schematic process flow diagram of yet another exemplary embodiment of the invention. Referring to Fig. 5, system 110 includes a distillative reaction system 40A with reactor sections 45 and 47, distillation sections 46 and 49, and reboiler B. System 110 also includes effluent separators 50A and 50B. The relationship of overhead effluent separator 50A and distillative reaction system 40A is similar to that discussed hereinabove with respect to Fig. 4A (system 108) and will not be repeated here.

In system 110, the liquid stream 43 from reboiler B of distillative reaction system 40A, is introduced into bottom effluent separator 50B, which has a first effluent E3 and a second effluent E4. The first effluent E3 is low-volatility feed and/or byproduct compounds that need to be purged. The second effluent E4 of the effluent separator 50B contains low-volatility compound(s) that are sought to be recycled for further reaction and are returned to the distillative reaction system 40A via F-1, F-2, F-3 and/or F-4.

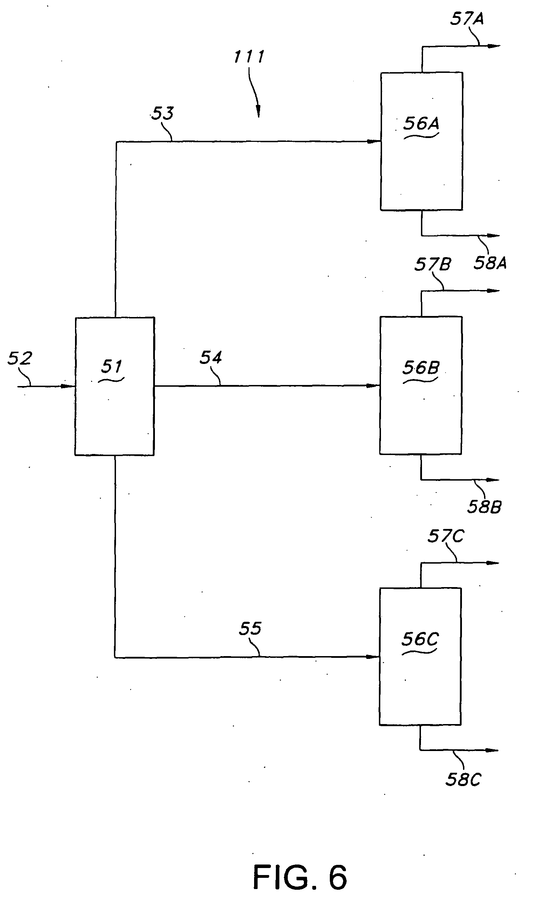

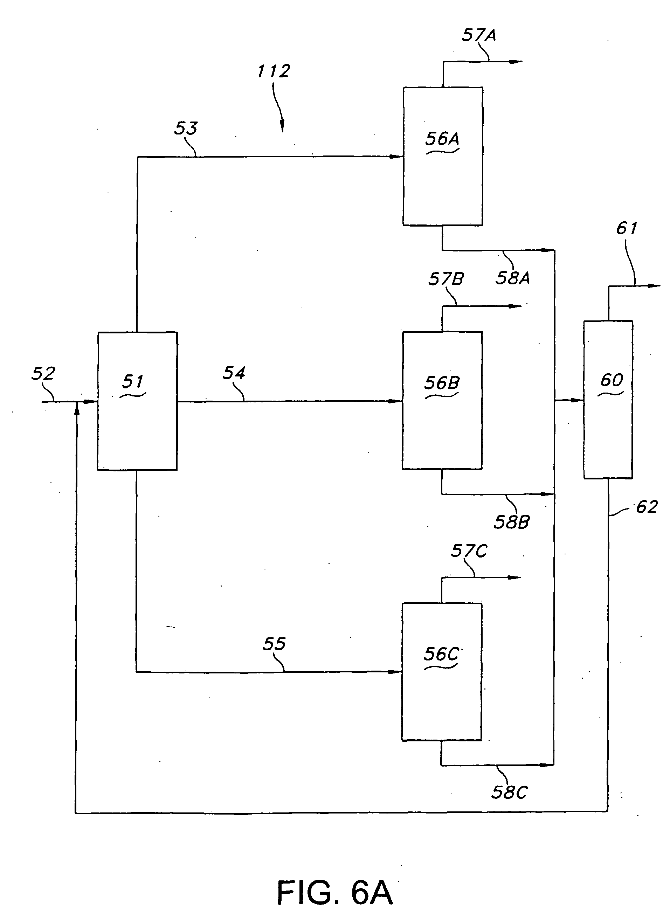

Referring now to Fig. 6, system 111 is shown separating naphtha into a plurality of constituents having different boiling ranges and isomerizing one or more of the constituents using one or more of the processes described hereinabove with respect to Figs. 1-5. In particular, a separator 51 is fed a stream containing naphtha via stream 52. Naphtha includes $C_5$ to $C_{10}$ hydrocarbon constituents or portions thereof. The plurality of constituents of the naphtha are drawn off the separator 51 via product streams 53, 54 and 55, each having a different composition and boiling range. The product streams 53, 54 and 55 are introduced into processors 56A, 56B and 56C, respectively. At least one or more of the processors of 56A, 56B and 56C uses one of the processes described above with respect to Figs. 1-5.

In accordance with another exemplary embodiment, the product streams 53, 54 and 55 include predominately $C_5$-$C_6$ constituents, $C_7$-$C_8$ constituents and $C_9$-$C_{10}$ constituents, respectively. In one embodiment, the product streams 53, 54 and 55 are introduced into the processors of 56A, 56B and 56C, respectively, where the processes are independently selected from any of the processes described above with respect to Figs. 1-5. In a second embodiment, at least one of the processors 56A, 56B and 56C is selected from one of the processors described above and the others are selected from any known conventional methods for the isomerization of either $C_5$-$C_6$, $C_7$-$C_8$ or $C_9$-$C_{10}$ constituents, or the other streams may be subjected to completely different processing (e.g., not isomerization) if suitable.

Referring now to Fig. 6A, in accordance with another exemplary embodiment, system 112 is similar to system 111, but further includes an effluent separator 60 having a first product stream 61 and a second product stream 62, as shown in Fig. 6A. The second product of the effluent separator 60 is introduced into the feed separator 51 via stream 62, for recycle to the processors 56A, 56B and 56C. Alternatively, each processor can have its own effluent separator(s) and recycle. Further alternatively, the effluent separator and the feed separator might be combined.

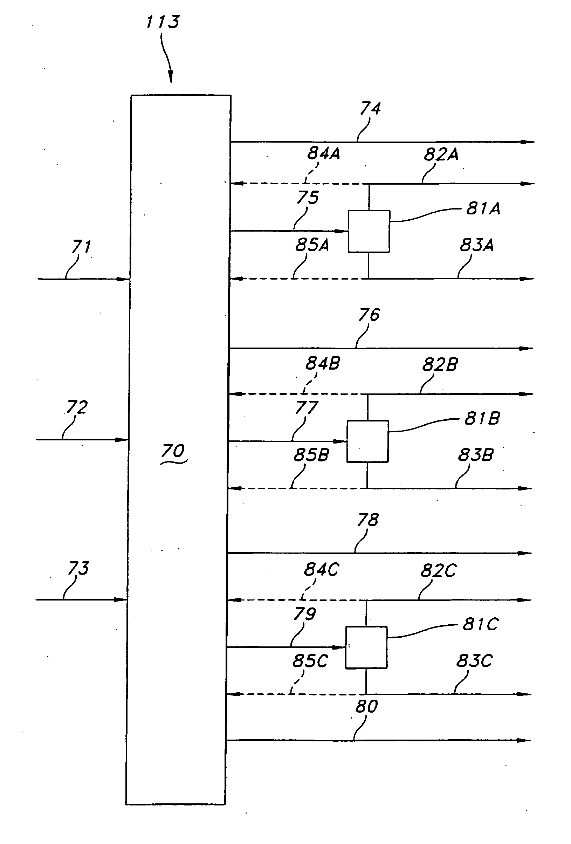

Referring now to Figure 7, system 113 depicts various options involving separation of feed compounds for sending to one or more distillative reaction unit(s). It includes at least one separator system 70. Separator system 70 may be a single separator unit or it may be a system comprising more than one separator, and may consist of separator(s) utilizing fractional distillation and/or physical separation, e.g., by adsorption or permeation. The feed to separator system 70 may be in a single stream or it may be in several streams, e.g., streams 71, 72, 73 as depicted in Figure 7. The separator system 70 produces at least two effluents. In Figure 7, system 70 is depicted as producing effluent streams, 74, 75,...80.

An example of separator system 70 is separation of a C5+ naphtha stream into, for example:

stream 74:   primarily pentane and isohexanes
stream 75:   primarily n-hexane
stream 76:   primarily cyclic C6's and C7 iso-paraffins
stream 77:   primarily n-heptane
stream 78:   primarily cyclic C7's and C8 iso-paraffins
stream 79:   primarily n-octane
stream 78:   primarily cyclic C8's and C9+compounds

It will be understood that such separation will not be absolute (i.e., some adjacent compounds will be present in each stream) and also, recognizing this, that such a separation can be accomplished by various possible approaches, including [a] distillation unit(s) alone; [b] distillation unit(s) followed by physical separation unit(s); and [c] physical separation unit(s) followed by distillation unit(s).

In the present embodiment of the invention, one or more of streams 74-80 is fed to one of the processes described hereinabove with respect to Figures 1-5. In Figure 7, it is depicted that streams 75, 77 and 79 would each be introduced into its own separate processor 81A, 81B and 81C, respectively, where the processes are independently selected from any of the processes described above with respect to Figures 1-5. Alternatively, it is not necessary that all of the processors 81A, 81B and 81C be as described above, as long as at least one is as so described. For example, the other processors could be selected from any known conventional methods for the isomerization of the constituents, or the streams may be subjected to completely different processing (e.g., not isomerization) if suitable.

In Figure 7, it is depicted that the effluents from the processors 81A, 81B and 81C are sent to further disposition, in streams 82A, 83A, 82B, 83B, 82C, and 83C. Referring to Figures 1-5, it will be understood that each of these streams is a net effluent stream following separation and/or combination within the processor units 81A, 81B and 81C.

As further options, any of the effluents from the processors 81A, 81B and 81C can be recycled in whole or in part to separator system 70. This is as depicted through streams 84A, 85A, 84B, 85B, 84C and/or 85C.

Yet another option related to alternatives for feed separation and combination is to combine two or more of the streams, e.g., streams 77 and 79, or a stream from another source, to feed to a distillative reaction unit processing the combined feeds.

## Claims

1. A process for the catalytic isomerization of a Paraffin selected from the group of linear or mono-branched $C_5$, $C_6$, $C_7$, $C_8$, $C_9$ $C_{10}$ and $C_{11}$ paraffins comprising:

   a) providing a distillative reaction system (10A, 40A) having

      (i) an uppermost reaction section (11, 47) including at least one catalyst bed positioned at a top portion of the distillative reaction system (10A, 40A), and
      (ii) a reboiler (B) and/or gas stripping section for vaporizing at least a portion (22, 42) of a bottom stream (21, 41) by the reboiler and/or the gas stripping section and returning the vaporized portion (22, 42) of the bottom stream to a bottom portion of the distillative reaction system (10A, 40A);

   b) introducing a hydrocarbon feed (F-1, F-2, F-3, F-4) including at least one linear or mono-branched $C_5$, $C_6$, $C_7$, $C_8$, $C_9$, $C_{10}$, and $C_{11}$ paraffin and a $H_2$ feed into the distillative reaction system (10A, 40A) below the uppermost reaction section (11. 47): and

   c) removing an overhead product stream (24, 48) from a portion of the distillative reaction system (10A, 40A) above the uppermost reaction section (11, 47), without substantial reflux meaning substantially none of the overhead product stream (24, 48) is returned to the top portion of the distillative reaction system (10A, 40A) nor is there any substantial cooling to cause condensation and internal reflux nor any substantial other liquid stream fed above said uppermost reaction section (11, 47).

   d) introducing the overhead product stream (24 48) into an effluent separator (25, 36, 50A), wherein the overhead product stream (24, 48) is separated into a first effluent (26, 35, E-1) containing the desired product and a second effluent (27, 37, E-2) containing unconverted and/or partially converted paraffins: and,

   e) introducing the second effluent (27, 37, E-2) of the effluent separator (25, 36, 50A) into the distillative reaction system (10A, 40A) at a position below the uppermost reaction section (11, 47).

2. The process of claim 1 further including introducing a gaseous reactant and/or stripping gas feed (F-1, F-2, F-3, F-4), preferably $H_2$ into the distillative reaction system (10A, 40A) at a position below the uppermost reaction section (11, 47).

3. The process of any preceding claim. wherein the distillative reaction system (40A) includes a distillation section (46).

4. The process of claim 3, wherein the distillative reaction system, (40A) includes one or more side streams (S-1) located at the distillation section (46).

5. The process of any preceding claim, wherein the distillative reaction system (40A) further includes a lower reaction section (45) including at least one catalyst bed.

6. The process of claims 3 and 5 wherein the distillation section (46) is disposed between the uppermost reaction section (47) and the lower reaction section (45).

7. The process of any preceding claim, wherein the distillative reaction system (10A, 40A) includes a gas stripping section (13) and/or a distillation section (46), which include one or more trays or packing.

8. The process of any preceding claim, wherein the distillative reaction system (10A) includes a gas stripping section (13) and the gaseous hydrocarbon feed F-1,F-2 is introduced in the distillative reaction system (10A), at a position below the gas stripping section (13).

9. The process of any preceding claim, further comprising
introducing the liquid portion (23, 43) of the bottom stream (21, 41) into an effluent separator (30, 36, 50B), wherein the liquid portion (23, 43) of the bottom stream (21, 41) is separated into a first product (31, 35, E-3) and a second product (32, 37, E-4); and, introducing the second product (32, 37, E-4) of the effluent separator (30, 36, 50B) into the distillative reaction system (10A, 40A,) at a position below the uppermost reaction section (11, 47).

**Patentansprüche**

1. Prozess für die katalytische Isomerisierung eines Paraffins gewählt aus der Gruppe von linearen oder monoverzweigten $C_5$-, $C_6$-, $C_7$-, $C_8$-, $C_9$-, $C_{10}$- und $C_{11}$-Paraffinen, welcher umfasst:

    a) Vorsehen eines destillativen Reaktionssystems (10A, 40A) mit
    i) einem obersten Reaktionsabschnitt (11, 47), der mindestens ein Katalysatorbett umfasst, das an einem oberen Teil des destillativen Reaktionssystems (10A, 40A) positioniert ist, und
    ii) einem Verdampfungsofen (B) und/oder einem Gasstrippabschnitt zum Verdampfen mindestens eines Teils (22, 42) eines unteren Stroms (21, 41) durch den Verdampfungsofen und/oder den Gasstrippabschnitt und zum Rückleiten des verdampften Teils (22, 42) des unteren Stroms zu einem unteren Teil des destillativen Reaktionssystems (10A, 40A);

    b) Einleiten einer Kohlenwasserstoffbeschickung (F-1, F-2, F-3, F-4), die mindestens ein lineares oder monoverzweigtes $C_5$-, $C_6$-, $C_7$-, $C_8$-, $C_9$-, $C_{10}$- und $C_{11}$-Paraffin umfasst, und einer $H_2$-Beschikkung in das destillative Reaktionssystem (10A, 40A) unter den obersten Reaktionsabschnitt (11, 47); und
    c) Entfernen eines oberen Produktstroms (24, 48) aus einem Teil des destillativen Reaktionssystems (10A, 40A) oberhalb des obersten Reaktionsabschnitts (11, 47) ohne wesentlichen Rückfluss, was bedeutet, dass im Wesentlichen nichts von dem oberen Produktstrom (24, 48) zu dem oberen Teil des destillativen Reaktionssystems (10, 10A, 40, 40A) zurückgeführt wird, und auch keine wesentliche Kühlung vorliegt, um Kondensation und internen Rückfluss zu bewirken, und auch kein wesentlicher anderer Produktstrom vorliegt, der oberhalb des obersten Reaktionsabschnitts (11, 47) eingespeist wird;
    d) Einleiten des oberen Produktstroms (24, 48) in einen Ablaufseparator (25, 36, 50A), wobei der obere Produktstrom (24, 48) in einen ersten Ablauf (26, 35, E-1), der das erwünschte Produkt enthält, und einen zweiten Ablauf (27, 37, E-2), der nicht umgewandelte und/oder teilweise umgewandelte Paraffine enthält, geteilt wird; und
    e) Einleiten des zweiten Ablaufs (27, 37, E-2) des Ablaufseparators (25, 36, 50A) in das destillative Reaktionssystem (10A; 40A) an einer Position unterhalb des obersten Reaktionsabschnitts (11, 47).

2. Prozess nach Anspruch 1, welcher weiterhin das Einleiten eines gasförmigen Reaktanten und/oder einer Strippgasbeschickung (F-1, F-2, F-3, F-4), vorzugsweise $H_2$, in das destillative Reaktionssystem (10A, 40A) an einer Position unterhalb des obersten Reaktionsabschnitts (11, 47) umfasst.

3. Prozess nach einem vorhergehenden Anspruch, **dadurch gekennzeichnet, dass** das destillative Reaktionssystem (40A) einen Destillationsabschnitt (46) umfasst.

4. Prozess nach Anspruch 3, **dadurch gekennzeichnet, dass** das destillative Reaktionssystem (40A) ein oder mehrere Seitenströme (S-1) umfasst, die sich an dem Destillationsabschnitt (46) befinden.

5. Prozess nach einem vorhergehenden Anspruch, **dadurch gekennzeichnet, dass** das destillative Reaktionssystem (40A) weiterhin einen unteren Reaktionsabschnitt (45) umfasst, der mindestens ein Katalysatorbett umfasst.

6. Prozess nach den Ansprüchen 3 und 5, **dadurch gekennzeichnet, dass** der Destillationsabschnitt (46) zwischen dem obersten Reaktionsabschnitt (47) und dem unteren Reaktionsabschnitt (45) angeordnet ist.

7. Prozess nach einem vorhergehenden Anspruch, **dadurch gekennzeichnet, dass** das destillative Reaktionssystem (10A, 40A) einen Gasstrippabschnitt (13) und/oder einen Destillationsabschnitt (46) umfasst, die ein oder mehre Böden oder Packung umfassen.

8. Prozess nach einem vorhergehenden Anspruch, **dadurch gekennzeichnet, dass** das destillative Reaktionssystem (10A) einen Gasstrippabschnitt (13) umfasst und die gasförmige Kohlenwasserstoffbeschickung (F-1, F-2) an einer Position unterhalb des Gasstrippabschnitts (13) in das destillative Reaktionssystem (10A) eingeleitet wird.

9. Prozess nach einem vorhergehenden Anspruch, welcher weiterhin umfasst:

Einleiten des flüssigen Teils (23, 43) des unteren Stroms (21, 41) in einen Ablaufseparator (30, 36, 50B), wobei der flüssige Teil (23, 43) des unteren Stroms (21, 41) in ein erstes Produkt (31, 35, E-3) und ein zweites Produkt (32, 37, E-4) unterteilt wird; und
Einleiten des zweiten Produkts (32, 37, E-4) des Ablaufseparators (30, 36, 50B) an einer Position unterhalb des obersten Reaktionsabschnitts (11, 47) in das destillative Reaktionssystem (10A, 40A).

**Revendications**

1. Procédé pour l'isomérisation catalytique d'une paraffine choisie dans le groupe des paraffines en $C_5$, $C_6$, $C_7$, $C_8$, $C_9$, $C_{10}$ et $C_{11}$, linéaires ou mono-ramifiées, consistant à :

a) disposer d'un système de réaction par distillation (10A, 40A) ayant

(i) une section réactionnelle la plus supérieure (11, 47) contenant au moins un lit de catalyseur, positionnée au niveau d'une partie supérieure du système de réaction par distillation (10A, 40A), et
(ii) un rebouilleur (B) et/ou une section de rectification au gaz pour évaporer au moins une partie (22, 42) d'un courant de fond (21, 41) par le rebouilleur et/ou la section de rectification au gaz et renvoyer la partie vaporisée (22, 42) du courant de fond vers une partie de fond du système de réaction par distillation (10A, 40A);

b) introduire une charge d'hydrocarbures (F-1, F-2, F-3, F-4) contenant au moins une paraffine en $C_5$, $C_6$, $C_7$, $C_8$, $C_9$, $C_{10}$ et $C_{11}$ linéaire ou mono-ramifiée et une charge de $H_2$ dans le système de réaction par distillation (10A, 40A) sous la section réactionnelle la plus supérieure (11, 47); et
c) retirer un courant de produit de distillat de tête (24, 48) à partir d'une partie du système de réaction par distillation (10A, 40A) au-dessus de la section réactionnelle la plus supérieure (11, 47) sans reflux substantiel, ce qui signifie qu'il n'y a pratiquement aucun courant de produit de distillat de tête (24, 48) qui est renvoyé vers la partie supérieure du système de réaction par distillation (10, 10A, 40, 40A), et qu'il n'y a non plus aucun refroidissement substantiel provoquant une condensation et un reflux interne, ni une quelconque charge substantielle d'autre courant liquide introduite au-dessus de ladite section la plus supérieure (11, 47);
d) introduire le courant de produit de distillat de tête (24, 48) dans un séparateur d'effluents (25, 36, 50A), le courant de produit de distillat de tête (24, 48) étant séparé en un premier effluent (26, 35, E-1) contenant le produit souhaité et un deuxième effluent (27, 37, E-2) contenant des paraffines non converties et/ou partiellement converties; et
e) introduire le deuxième effluent (27, 37, E-2) du séparateur d'effluents (25, 36, 50A) dans le système de réaction par distillation (10A, 40A) en une position se trouvant sous la section réactionnelle la plus supérieure (11, 47).

2. Procédé selon la revendication 3, comprenant en outre l'introduction d'un réactif gazeux et/ou d'une charge de gaz de rectification (F-1, F-2, F-3, F-4), de préférence $H_2$, dans le système de réaction par distillation (10A, 40A) en une position se trouvant sous la section réactionnelle la plus supérieure (11, 47).

3. Procédé selon l'une quelconque des revendications précédentes, dans lequel le système de réaction par distillation (40A) comprend une section de distillation (46).

4. Procédé selon la revendication 3, dans lequel le système de réaction par distillation (40A) comprend un ou plusieurs courants latéraux (S-1) situés au niveau de la section de distillation (46).

5. Procédé selon l'une quelconque des revendications précédentes, dans lequel le système de réaction par distillation (40A) comprend en outre une section réactionnelle inférieure (45) contenant au moins un lit de catalyseur.

6. Procédé selon les revendications 3 et 5, dans lequel la section de distillation (46) est disposée entre la section réactionnelle la plus supérieure (47) et la section réactionnelle inférieure (45).

7. Procédé selon l'une quelconque des revendications précédentes, dans lequel le système de réaction par distillation (10A, 40A) comprend une section de rectification au gaz (13) et/ou une section de distillation (46) qui comprennent un ou plusieurs plateaux ou garnitures.

8. Procédé selon l'une quelconque des revendications précédentes, dans lequel le système de réaction par distillation (10A) comprend une section de rectification au gaz (13) et la charge d'hydrocarbures gazeux (F-1, F-2) est introduite dans le système de réaction par distillation (10A) en une position se trouvant sous la section de rectification au gaz (13).

9. Procédé selon l'une quelconque des revendications précédentes, comprenant en outre l'introduction de la partie liquide (23, 43) du courant de fond (21, 41) dans un séparateur d'effluents (30; 36, 50B), la partie liquide (23, 43) du courant de fond (21, 41) étant séparée en un premier produit (31, 35, E-3) et un deuxième produit (32, 37, E-4); et l'introduction du deuxième produit (32, 37, E-4) du séparateur d'effluents (30, 36, 50B) dans le système de réaction par distillation (10A, 40A) en une position se trouvant sous la section réactionnelle la plus supérieure (11, 47).

*101*

*24*

*10*

*11*

F-1

F-2

*22*

*B*

*21*

*23*

# FIG. 1

FIG. 1A

FIG. 1B

**FIG. 2**

FIG. 3

**FIG. 3A**

**FIG. 4**

**FIG. 4A**

FIG. 4B

**FIG. 5**

FIG. 6

FIG. 6A

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10243444 A
- WO 0015319 A
- US 6767859 B